# EUROPEAN PATENT APPLICATION

(11) **EP 4 474 943 A1**
(43) Date of publication of application: **11.12.2024**
(21) Application number: 24700665.3
(22) Date of filing: 19.01.2024
(51) Int. Cl.: G06F 1/16, A61B 5/00, G06F 3/01

(54) **WEARABLE ELECTRONIC DEVICE INCLUDING BATTERY**

(30) Priority: 27.04.2023 KR 20230055484; 24.05.2023 KR 20230067008
(71) Applicant: Samsung Electronics Co., Ltd., Suwon-si, Gyeonggi-do 16677 (KR)
(72) Inventor: PARK, Haekeu, Suwon-si, Gyeonggi-do 16677 (KR); RHEE, Daesun, Suwon-si, Gyeonggi-do 16677 (KR)
(74) Representative: HGF
(86) International application number: PCT/KR2024/000982
(87) International publication number: WO 2024/225576

(57) **Abstract**

A wearable electronic device according to an embodiment of the disclosure may comprise a ring-shaped frame. The wearable electronic device may comprise a battery seated on the frame. The wearable electronic device may comprise an electronic component electrically connected to the battery. The wearable electronic device may comprise a molding member integrally coupled to the frame to surround the battery and the electronic component. The wearable electronic device may comprise a compression member disposed to contact the battery and configured so that a part thereof, in contact with an expanding portion of the battery, is compressible when the battery swells. The compression member may be positioned between the frame and the molding member.

## Description

### [TECHNICAL FIELD]

Various embodiments of the disclosure relate to a wearable electronic device having a battery and a molding member surrounding a space around the battery.

### [BACKGROUND ART]

Wearable electronic devices are devices, such as clothes, watches, or glasses, which may be freely worn on the user's body. Wearable electronic devices may be classified into various types, such as smart glasses, smart watches, and ring-type wearable electronic devices (or smart rings), depending on their shape.

Among wearable electronic devices, the ring-shaped wearable electronic device is worn on the user's body part (eg., a finger) through a ring formed therein. A smart ring may be able to detect the user's biometric signals through various sensors included therein when worn by the user, analyze the user's biometric signals detected, and provide the user with health information using analysis data, such as the user's pulse, sleep level, blood pressure, etc.

### [DISCLOSURE]

### [TECHNICAL PROBLEM]

A ring-shaped wearable electronic device may include a frame configuring the skeleton (or framework) of the wearable electronic device, a battery configured to supply power to electronic components provided in the wearable electronic device, and a molding member coupled to the frame to configure an outer portion or inner portion of the wearable electronic device.

In an electronic device including a battery, the battery may swell due to a change in external environment, e.g., temperature and repeated use of the device. Such battery swelling may deform the structure of the electronic device. In particular, in an electronic device having at least a portion of the space around the battery, formed of a molding member, e.g., a ring-shaped wearable electronic device, battery swelling may damage the molding member due to volume expansion of the battery or reduce the inner diameter of the ring-shaped wearable electronic device, causing it difficult to pull out the user's finger.

### [TECHNICAL SOLUTION]

Various embodiments of the disclosure may provide a ring-shaped wearable electronic device including a compression member disposed near the battery to receive the volume expansion of the battery that occurs when the battery swells.

According to an embodiment of the disclosure, a wearable electronic device may comprise a ring-shaped frame. The wearable electronic device may comprise a battery seated on the frame. The wearable electronic device may comprise an electronic component electrically connected to the battery. The wearable electronic device may comprise a molding member integrally coupled to the frame to surround the battery and the electronic component. The wearable electronic device may comprise a compression member disposed to contact the battery and configured so that a part thereof, in contact with an expanding portion of the battery, is compressible when the battery swells. The compression member may be positioned between the frame and the molding member.

According to various embodiments of the disclosure, even when volume expansion occurs due to swelling of the battery, the compression member disposed near the battery may be compressed to receive the expanded volume of the battery, preventing damage to the product and human accidents.

According to various embodiments of the disclosure, particularly for ring-shaped wearable electronic devices in which the space around the battery is filled with a molding member, product deformation and resultant accidents may be prevented by a compression member disposed near the battery to receive the expanded volume of the battery due to swelling.

Effects of the disclosure are not limited to the foregoing, and other unmentioned effects would be apparent to one of ordinary skill in the art from the following description. In other words, unintended effects in practicing embodiments of the disclosure may also be derived by one of ordinary skill in the art from the embodiments of the disclosure.

### [DESCRIPTION OF DRAWINGS]

The above and other aspects, features, and advantages of certain embodiments of the disclosure will be more apparent from the following description taken in conjunction with the accompanying drawings, in which:
FIG. 1 is a view illustrating an electronic device in a network environment according to various embodiments;
FIG. 2 is a perspective view illustrating a ring-shaped wearable electronic device according to an embodiment of the disclosure;
FIG. 3 is a perspective view illustrating an inside of a ring-shaped wearable electronic device according to an embodiment of the disclosure;
FIG. 4 is a cross-sectional view illustrating a half part of a ring-shaped wearable electronic device according to an embodiment of the disclosure;
FIG. 5 is a functional block diagram illustrating a ring-shaped wearable electronic device according to an embodiment of the disclosure;
FIG. 6A is a view schematically illustrating an arrangement structure of some components provided inside a ring-shaped wearable electronic device according to an embodiment of the disclosure;
FIG. 6B is a view schematically illustrating an arrangement structure of some components provided inside a ring-shaped wearable electronic device according to an embodiment of the disclosure;
FIG. 6C is a view schematically illustrating an arrangement structure of some components provided inside a ring-shaped wearable electronic device according to an embodiment of the disclosure;
FIG. 7 is a perspective view illustrating an arrangement structure between some components provided inside a ring-shaped wearable electronic device according to an embodiment of the disclosure;
FIG. 8 is a view schematically illustrating a change in volume of a battery and a compression member when a battery swells in a ring-shaped wearable electronic device according to an embodiment of the disclosure; and
FIG. 9 is a plan view illustrating a structure of a battery of a ring-shaped wearable electronic device according to an embodiment of the disclosure.

### [MODE FOR CARRING OUT THE INVENTION]

It should be appreciated that various embodiments of the disclosure and the terms used therein are not intended to limit the technological features set forth herein to particular embodiments and include various changes, equivalents, or replacements for a corresponding embodiment.

With regard to the description of the drawings, similar reference numerals may be used to refer to similar or related elements.

It is to be understood that a singular form of a noun corresponding to an item may include one or more of the things, unless the relevant context clearly indicates otherwise.

As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases.

As used herein, such terms as "1st" and "2nd," or "first" and "second" may be used to simply distinguish a corresponding component from another, and does not limit the components in other aspect (e.g., importance or order).

It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

It will be further understood that the terms "comprise" and/or "have," as used herein, specify the presence of stated features, integers, steps, operations, elements, and/or components, but do not preclude the presence or addition of one or more other features, integers, steps, operations, elements, components, and/or groups thereof.

It will be understood that when a component is referred to as "connected to," "coupled to", "supported on," or "contacting" another component, the components may be connected to, coupled to, supported on, or contact each other directly or via a third component.

Throughout the specification, when one component is positioned "on" another component, the first component may be positioned directly on the second component, or other component(s) may be positioned between the first and second component.

The term "and/or" may denote a combination(s) of a plurality of related components as listed or any of the components.

Hereinafter, the working principle and embodiments of the disclosure are described with reference to the accompanying drawings.

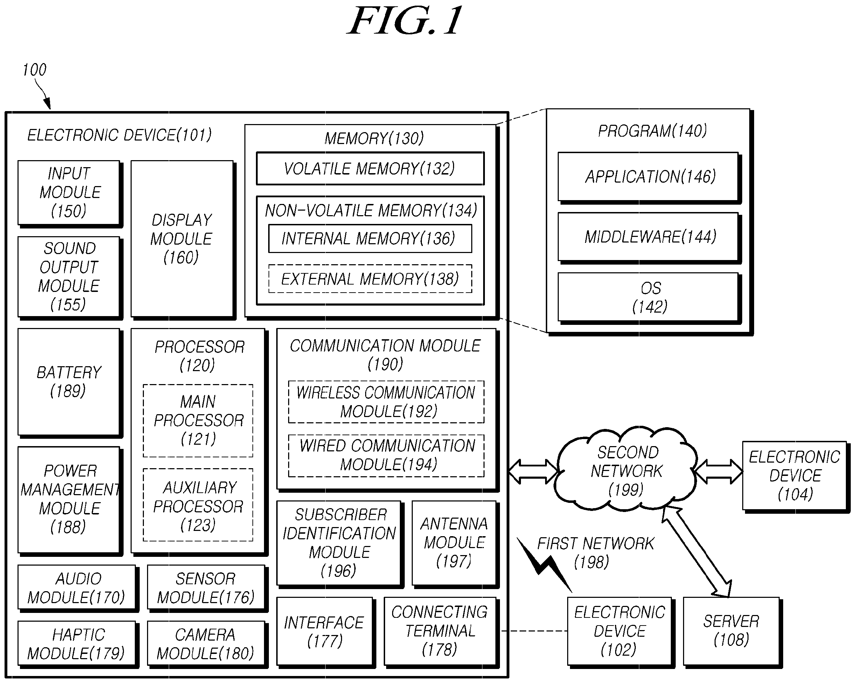

FIG. 1 is a block diagram illustrating an electronic device 101 in a network environment 100 according to various embodiments.

Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display 160 may include, for example, a display, a hologram device, or a projector and control circuitry to control a corresponding one of the display, hologram device, and projector. According to an embodiment, the display 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductive body or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or health-care) based on 5G communication technology or IoT-related technology.

The electronic device according to various embodiments of the disclosure may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

The following description focuses primarily on a ring-shaped wearable electronic device according to embodiments of the disclosure, but the disclosure is not limited thereto. Various embodiments of the disclosure may be applied to electronic devices of various configurations and types, including ring-shaped wearable devices.

In an embodiment, the ring-shaped wearable electronic device of the disclosure may detect the user's biometric signals through various sensors. In some embodiments, the ring-shaped wearable electronic device may analyze the detected biometric signal and may wirelessly transmit analysis data such as pulse, sleep level, blood pressure, or the like to a preset external device (e.g., user equipment having a display, a monitor, a TV, or the like) to provide the analysis data in the form of a text, a number, or an image to the user. Further, in some embodiments, the ring-shaped wearable electronic device according to the disclosure may separately include a display, and in this case, the analysis data in the form of a text, a number, or an image may be provided to the user through the display.

Hereinafter, a configuration of a ring-shaped wearable electronic device according to an embodiment of the disclosure is described in detail with reference to the accompanying drawings.

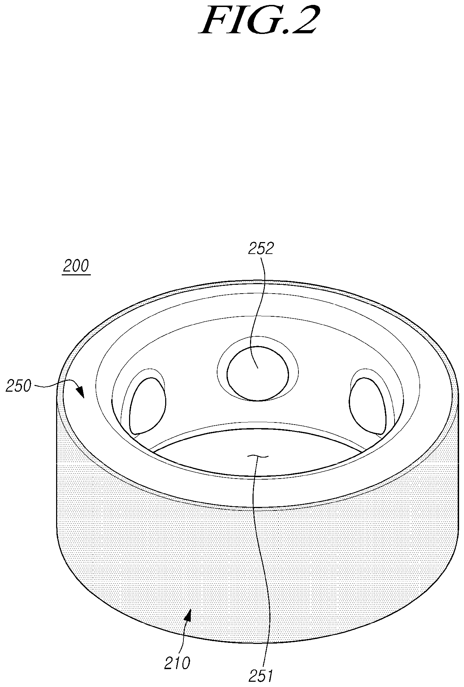

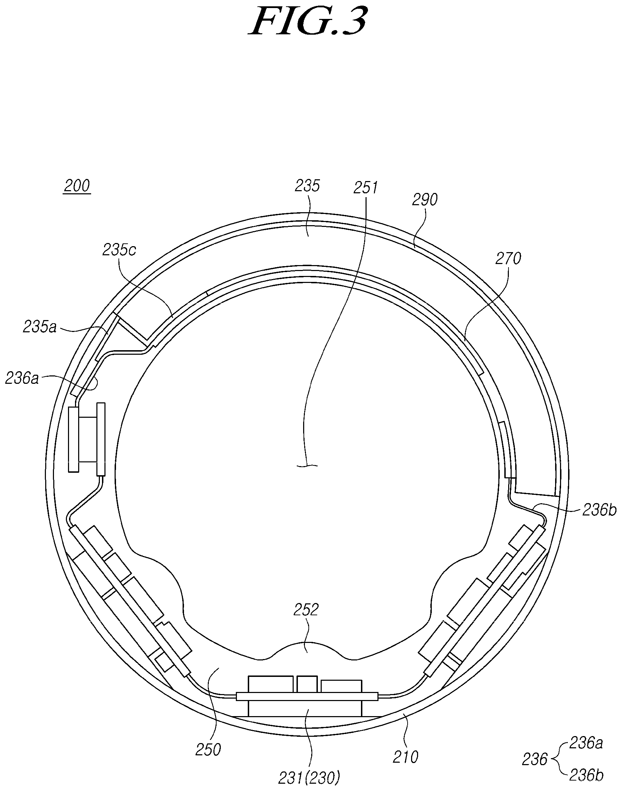

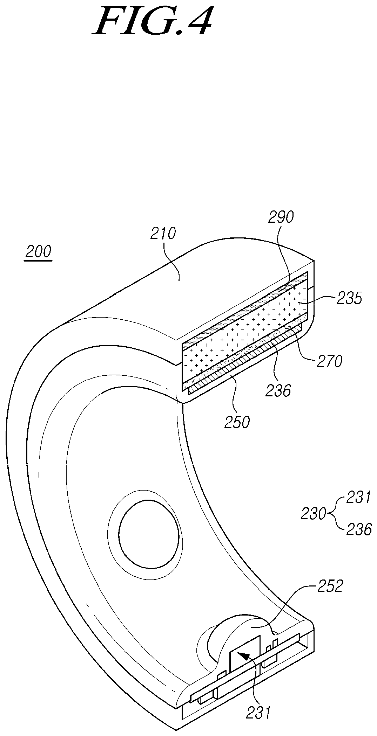

FIG. 2 is a perspective view illustrating a ring-shaped (or, in other words, annular) wearable electronic device according to an embodiment of the disclosure. FIG. 3 is a perspective view illustrating an inside of a ring-shaped wearable electronic device according to an embodiment of the disclosure. FIG. 4 is a cross-sectional view illustrating a half part of a ring-shaped wearable electronic device according to an embodiment of the disclosure.

Referring to FIGS. 2 to 4, a wearable electronic device 200 (e.g., the electronic device 101 of FIG. 1) according to an embodiment may overall have a ring shape to be detachable from the user's finger. For example, the wearable electronic device 200 may be provided in a circular shape, but the disclosure is not limited thereto.

According to an embodiment, the wearable electronic device 200 may include a frame 210 (which may also be described as: a support; a support portion, member, body, or component; an outer portion, member, body, or component; a perimeter or peripheral portion, member, body, or component; an outer housing; or an outer housing portion, member, body, or component), an electrical circuit part 230 (which may also be described as an electrical circuit portion, component, or module), a member 250 (which may also be described as: a molding member; a molded member; an inner member; an inner housing; or an inner housing portion, member, body, or component) , a compression member 270 (which may also be described as a compressible, or resilient, member, body, component, or element), and an adhesive member 290 (or adhesive body, component, or element).

According to an embodiment, the wearable electronic device 200 may include a frame 210. According to an embodiment, the frame 210 may constitute the skeleton (or supporting framework, or support structure) of the wearable electronic device 200. According to an embodiment, the frame 210 may form an outer portion of the wearable electronic device 200. In an embodiment, the frame 210 may be formed of a metal material. For example, the frame 210 may be formed of a highly rigid titanium material, but the disclosure is not limited thereto. The frame may be annular.

According to an embodiment, the wearable electronic device 200 may include a molding (or molded) member 250. According to an embodiment, e.g., as illustrated in FIG. 2, the molding member 250 may be coupled to the frame 210 inside the frame 210 (specifically, the inner circumferential surface of the frame 210) to form the inner portion of the wearable electronic device 200. In this case, the frame 210 may be referred to as an outer ring member (or outer annular member), and the molding member 250 may be referred to as an inner ring member (or inner annular member).

With reference to FIGS. 2 to 4, it is illustrated and described that the frame 210 and the molding member 250 form the outer portion and the inner portion, respectively, of the wearable electronic device 200, but the disclosure is not limited thereto. According to another embodiment of the disclosure, the frame 210 may form an inner portion of the wearable electronic device 200. For example, unlike that illustrated in FIG. 2, when the molding member 250 is coupled to the frame 210 outside the frame 210 (specifically, the outer circumferential surface of the frame 210), the frame 210 may form an inner portion of the wearable electronic device. In this case, the frame 210 may be referred to as an inner ring member, and the molding member 250 may be referred to as an outer ring member.

In an embodiment, as illustrated in FIG. 2, a hole 251 into which the user's finger is inserted may be provided inside the molding member 250. In an embodiment, the diameter of the hole 251 of the molding member 250 may be appropriately set according to the thickness of the user's finger.

According to an embodiment, the wearable electronic device 200 may include an electrical circuit part 230. In an embodiment, the electrical circuit part 230 may be positioned between the frame 210 and the molding member 250 (in other words, the electrical circuit portion 230 may be housed in a volume defined by the frame 210 and the member 250). According to an embodiment, each component of the electrical circuit part 230 may be disposed on the inner circumferential surface side of the frame 210. According to an embodiment, the electrical circuit part 230 may include one or more electronic elements configured to support various functions of the wearable electronic device 100. According to an embodiment, the electrical circuit part 230 may include at least one electronic component (e.g., the printed circuit board 236) electrically connected to the battery 235 to be described below.

According to an embodiment, the electrical circuit part 230 may include a battery 235 for supplying power required for each electronic element of the wearable electronic device 200, and one or more printed circuit boards (PCBs) 236 on which various electronic elements configured to support various functions are disposed.

According to an embodiment, the battery 235 may be configured to supply power to each electronic element (or electronic component) provided in the wearable electronic device 200. In other words, the battery may power operation of the device 200.

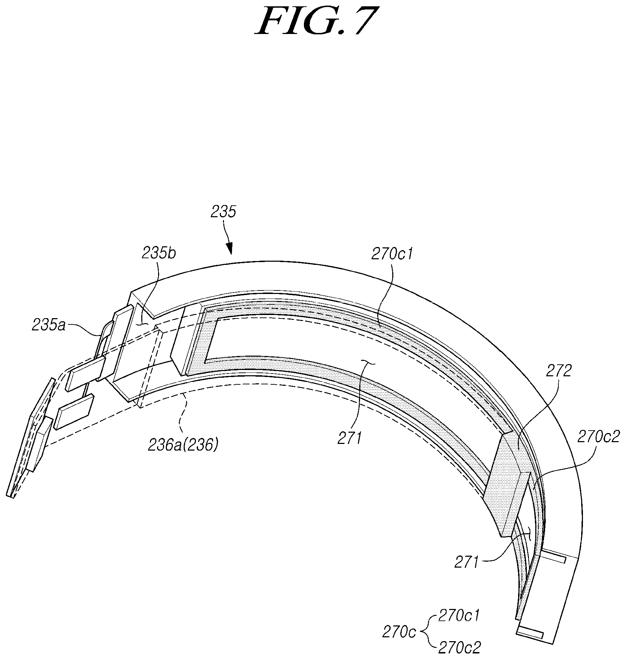

In an embodiment, a connector 235a for electrical connection (or access) to the electronic component (e.g., the printed circuit board 236) may be provided on one side of the battery 235. In an embodiment, an escape space (e.g., the escape space 235b of FIG. 7) for extending an electrode of the connector 235a may be formed in a portion of the battery 235 in which the connector 235a is provided.

In an embodiment, the battery 235 may include a supporting member 235c coupled to the battery 235 to cover the escape space 235b from the outside of the battery 235. In an embodiment, the supporting member 235c may have a block shape as a whole. In this case, the supporting member 235c may be received in the escape space 235b.

In an embodiment, one surface (e.g., a surface facing the hole 251) of the supporting member 235c may contact one side portion 236a of the one or more printed circuit boards 236.

In an embodiment, the supporting member 235c may be formed of substantially the same material as the compression member 270. In an embodiment, the supporting member 235c may be formed of a flexible polyurethane foam, but the disclosure is not limited thereto. The supporting member 235c may be formed of, e.g., at least one of rubber (natural rubber or synthetic rubber), elastomer, chloroprene, silicone, or neoprene.

In an embodiment, the escape space 235b adjacent to the connector 235a of the battery 235 may be a substantially narrow space, and the battery 235 may not include a separate supporting member 235c as illustrated in FIG. 7.

In an embodiment, the battery 235 may be implemented as a secondary battery capable of charging and discharging, but the disclosure is not limited thereto. The battery 235 may correspond to, e.g., a lithium ion battery.

In an embodiment, various electronic elements and electrical circuits including one or more sensors may be disposed on the one or more printed circuit boards 236. According to an embodiment, the one or more printed circuit boards 236 of the electrical circuit part 230 may include one or more flexible printed circuit boards (FPCBs) configured to be electrically connectable to the battery 235 inside the wearable electronic device 200 having a curved structure. For example, the FPCB 236 may be electrically connected to the connector 235a of the battery 235, and power from the battery 235 may be supplied to various electronic components provided in the electrical circuit part 230 through the FPCB 236. In an embodiment, the FPCB 236 may be provided with a charging circuit for wireless charging of the battery 235.

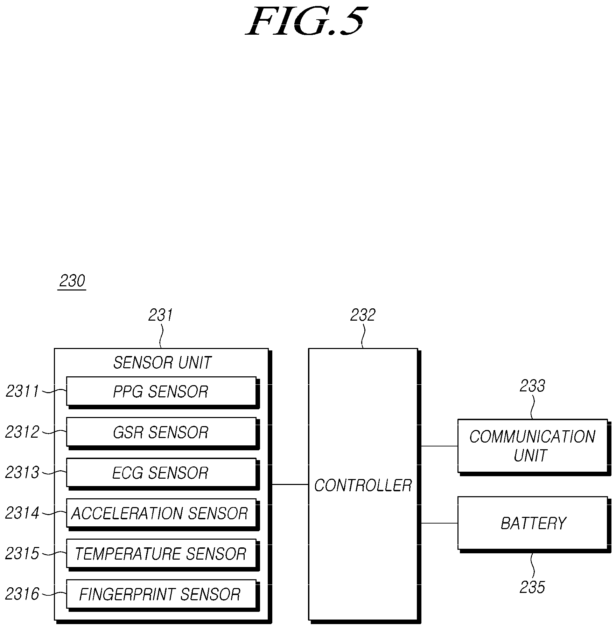

According to an embodiment, the electrical circuit part 230 may include at least one sensor unit 231 for detecting the user's biometric signal. According to an embodiment, the electrical circuit part 230 may include various electronic components for various functions of the wearable electronic device 200 in addition to the one or more sensor units 231 for biometric signal detection. The electrical circuit part 230 may include various electronic components, such as a control module (e.g., the controller 232), a communication module (e.g., the communication unit 233), an audio module (not shown), a power module (e.g., the battery 235), and an input module (not shown). The configuration of the electrical circuit part 230 is described below in more detail with reference to FIG. 5.

According to an embodiment, the battery 235 may be disposed on the side of one surface (e.g., the inner circumferential surface) of the frame 210. In an embodiment, the battery 235 may be fixed to the inner circumferential surface of the frame 210 by a bonding method. In an embodiment, the battery 235 may be attached to the inner circumferential surface of the frame 210 by the adhesive member 290 interposed between the battery 235 and the frame 210, but the disclosure is not limited thereto. In an embodiment, the adhesive member 290 may include a tape having two opposite surfaces coated with an adhesive material (e.g., acrylic).

According to an embodiment, the battery 235 may have a bent (or curved) shape. In an embodiment, the battery 235 may be bent to have a curvature substantially corresponding to the curvature of the inner circumferential surface of the frame 210 to which the battery 235 is attached. However, the disclosure is not limited thereto.

According to an embodiment, the molding member 250 may be coupled to the frame 210 to overall cover one surface (e.g., the inner circumferential surface of the frame 210) of the frame 210 and the electrical circuit part 230 and the battery 235 in the state in which the electrical circuit part 230 (e.g., the battery 235 and the PCB 236) is disposed on the side of the inner circumferential surface of the frame 210. In an embodiment, the molding member 250 may be coupled with the frame 210 by at least one of an injection process or a molding process. In an embodiment, the molding member 250 may be formed of a synthetic resin having a transparent material, but the disclosure is not limited thereto. The molding member 250 may be formed of, e.g., an epoxy resin.

According to an embodiment, at least one biometric signal detector 252 protruding toward the inside of the hole 251, e.g., the center of the hole 251, may be provided on the inner circumferential surface of the molding member 250. In other words, in certain embodiments, the device 200 comprises at least one detector or sensor protruding, or extending, radially inwards from a surface of the inner portion of the device (which may, for example, be the molded member 250 or the frame 210, depending on the configuration of the device). In an embodiment, the number of biometric signal detectors 252 may correspond to the number of sensor units 231 of the electric circuit part 230 described above, but the disclosure is not limited thereto. In an embodiment, a corresponding sensor unit 231 may be positioned inside a plurality of biometric signal detectors 252. In this case, when the user wears the wearable electronic device 200, the plurality of biometric signal detectors 252, respectively, may contact the user's fingers, and may detect each of the user's body signals through the corresponding sensor unit 231.

According to an embodiment, the wearable electronic device 200 may include a compression (or compressible) member 270 disposed adjacent to the battery 235. In an embodiment, the compression member 270 may be disposed to contact at least one surface of the battery 235. In an embodiment, the compression member 270 may be attached to at least one surface of the battery 235. For example, as illustrated in FIGS. 3 and 4, the compression member 270 may be attached to an inner surface (e.g., a surface facing the inside of the hole 251, or, in other words, a surface facing radially inwards) of the battery 235.

According to an embodiment, swelling, i.e., volume expansion of the battery 235, may occur in the battery 235 of the wearable electronic device 200 as the internal electrolyte vaporizes due to changes in the external environment such as temperature, and repeated use. According to an embodiment, the compression member 270 disposed to contact at least one surface of the battery 235 may be configured to be compressible through contact with an expanding portion of the swollen battery 235 when the battery 235 swells. In an embodiment, when the battery 235 gradually swells more and more, the compression member 270 may be gradually compressed more and more by the swollen portion.

In an embodiment, the compression member 270 may be formed of a flexible polyurethane foam, but the disclosure is not limited thereto. The compression member 270 may be formed of, e.g., at least one of rubber (natural rubber or synthetic rubber), elastomer, chloroprene, silicone, or neoprene.

In an embodiment, the compression member 270 may have a compression rate that is substantially the same as or higher than the volume expansion rate of the battery 235 that expands during battery swelling. In an embodiment, the thickness of the compression member 270 may be set so that a value obtained by multiplying the thickness of the compression member 270 by the compression rate of the compression member 270 is equal to or greater than a value obtained by multiplying the thickness of the battery 235 by the volume expansion rate of the battery 235. In this case, the expanding portion due to swelling of the battery 235 may be received by the corresponding compression of the compression member 270. Due to the compression member 270, the pressure applied to the molding member 250 due to the volume expansion of the battery 235 may be reduced, and interference between the battery 235 and the molding member 250 may be reduced. In other words, the compressible member may be able to deform (compress) to accommodate an increase in battery volume (e.g. battery swelling), without deforming the molded member 250 and so without reducing the size (e.g. cross-sectional area) of the hole 251.

In an embodiment, two opposite surfaces of the compression member 270 may be coated with an adhesive material. In this case, the compression member 270 may be attached and fixed to each counterpart (e.g., the frame 210, the battery 235, or the PCB 236) contacting the compression member 270. Here, two opposite surfaces of the compression member 270 may refer to a surface facing the center of the wearable electronic device 200 (e.g., the center of the hole 251 of the molding member 250) (in other words, a surface facing radially inwards) and a surface facing away from the center of the wearable electronic device 200 (in other words, a surface facing radially outwards). According to an embodiment, one surface of the compression member 270 may be fixedly disposed in contact with the battery 235 as described above, while the other surface may be fixedly disposed in contact with the PCB 236 (e.g., FPCB) of the electrical circuit part 230 as illustrated in FIGS. 3 and 4.

FIG. 5 is a functional block diagram illustrating a ring-shaped wearable electronic device according to an embodiment.

FIG. 5 is a block diagram illustrating a state in which a sensor unit 231, a battery 235, and a communication unit 233 are electrically connected to a controller 232 according to an embodiment.

Referring to FIG. 5, the electrical circuit part 230 according to an embodiment may include a sensor unit (or module) 231, a communication unit (or module) 233, a battery 235 (or battery unit, pack, or module), and a controller 232 (or control unit or module).

According to an embodiment, the sensor unit 231 may be configured to perform various functions. In an embodiment, the sensor unit 231 may perform a function of detecting whether the user has apnea during sleep, a function of detecting whether the user is asleep and a sleep stage, a function of measuring blood pressure, a function of measuring calorie consumption, a function of measuring skin hydration, a function of authenticating the user, and/or a function of measuring temperature, and a touch input function for controlling the device. In an embodiment, the sensor unit 231 may include at least one of various sensors to perform the above-described various functions.

According to an embodiment, the sensor unit 231 may include a photoplethysmography (PPG) sensor 2311 for detecting whether the user has apnea during sleep. In an embodiment, the PPG sensor 2311 may measure the user's blood oxygen saturation to detect whether the user has apnea during sleep.

According to an embodiment, the PPG sensor 2311 may include a light emitter (not shown) configured to emit light toward the user's finger when the user wears the wearable electronic device 200, and a light receiver (not shown) configured to receive light reflected from the blood vessel inside the user's finger among the light emitted from the light emitter. In an embodiment, the light emitter may include at least one light source. For example, the at least one light source may include at least one of a red light emitting diode (LED) or an infrared LED. In an embodiment, the light receiver may include at least one photodiode.

According to an embodiment, the sensor unit 231 may include a galvanic skin response sensor (GSR) 2312 for detecting a change in current due to moisture discharged from the user's finger skin. In an embodiment, the GSR sensor 2312 may be configured to detect a change in skin humidity due to the sympathetic nervous system of the body as a change in electrical resistance. In this case, the controller 232 may determine whether the current state of the user is an awakened state or a drowsy state based on the change in the resistance of the GSR sensor 2312 corresponding to the change in the skin humidity, and determine whether the user sleeps. For example, the controller 232 may determine whether the user is drowsy by simultaneously measuring the skin impedance response (SIR) corresponding to the alternating current (AC) component and the overall skin impedance level (SIL) corresponding to the direct current (DC) component, using the signal of the GSR sensor 2312. Specifically, when the user is dozing off, the expression interval of the SIR becomes longer, the expression frequency decreases, and the SIL increases.

According to an embodiment, the sensor unit 231 may include an electrocardiogram (ECG) sensor 2313. In an embodiment, the ECG sensor 2313 may be configured to measure the user's heartrate and constantness. In an embodiment, the ECG sensor 2313 may be used to check the size and position of the user's heart or whether the user's heart is damaged. For example, the ECG sensor 2313 may be used to measure and diagnose an abnormal rhythm of the heart. In particular, the ECG sensor 2313 is effective in measuring an abnormal rhythm due to damage to conductive tissue that transmits an electrical signal.

According to an embodiment, the sensor unit 231 may include an acceleration sensor 2314 for detecting the user's movement. The acceleration sensor 2314 may correspond to, e.g., a three-axis acceleration sensor that measures acceleration in the X-axis, Y-axis, and Z-axis directions. In an embodiment, the acceleration sensor 2314 may be replaced by a gyro sensor (not shown) or may detect the user's movement in combination with the gyro sensor.

According to an embodiment, the sensor unit 231 may include a temperature sensor 2315 for detecting the user's body temperature or an object's temperature. In an embodiment, the temperature sensor 2315 may correspond to a contactless temperature sensor, but the disclosure is not limited thereto.

According to an embodiment, the sensor unit 231 may include a fingerprint sensor 2316 for detecting the user's fingerprint. The fingerprint sensor 2316 may be implemented by, e.g., an optical fingerprint recognition method, a capacitive fingerprint recognition method, or an ultrasonic fingerprint recognition method, but the disclosure is not limited thereto.

According to an embodiment, the controller 232 may be configured to perform the overall operation of the wearable electronic device 200.

In an embodiment, the controller 232 may measure the blood pressure of the user by using a known blood pressure estimation method based on the ECG signal detected by the ECG sensor 2313 and the blood oxygen saturation detected by the PPG sensor 2311.

In an embodiment, the controller 232 may measure the calorie consumption of the user based on the signal detected by the acceleration sensor 2314.

In an embodiment, the controller 232 may detect the sleep stage of the user by using the signal detected by the PPG sensor 2311 or the ECG sensor 2313 and the signal detected by the acceleration sensor 2314.

In an embodiment, the controller 232 may perform the user authentication function by using signals detected through the fingerprint sensor 2316 and the ECG sensor 2313. For example, the controller 232 may perform user authentication by comparing the detected fingerprint data and ECG data with the user's fingerprint data and ECG data previously stored in memory (not shown).

In an embodiment, the controller 232 may receive power from the battery 235 and may supply the received power to the sensor unit 231 or the communication unit 233.

According to an embodiment, the communication unit 233 may be configured to transmit various signals detected by the sensor unit 231 to an external device (e.g., the electronic device 102 of FIG. 1) or to receive signals transmitted from the external device 102.

According to an embodiment, the communication unit 233 may include an element that performs a wireless communication function of any one of Bluetooth, Wi-Fi, and Zigbee technologies. In an embodiment, the communication unit 233 may include a near-field communication (NFC) chip for implementing a mobile payment function.

According to an embodiment, the battery 235 may wirelessly receive power from an external wireless charging device (not shown) through the wireless charging circuit and be charged with the power. However, the disclosure is not limited to this, and the battery 235 may be electrically connected to a separate external charger to be charged in a wired charging manner.

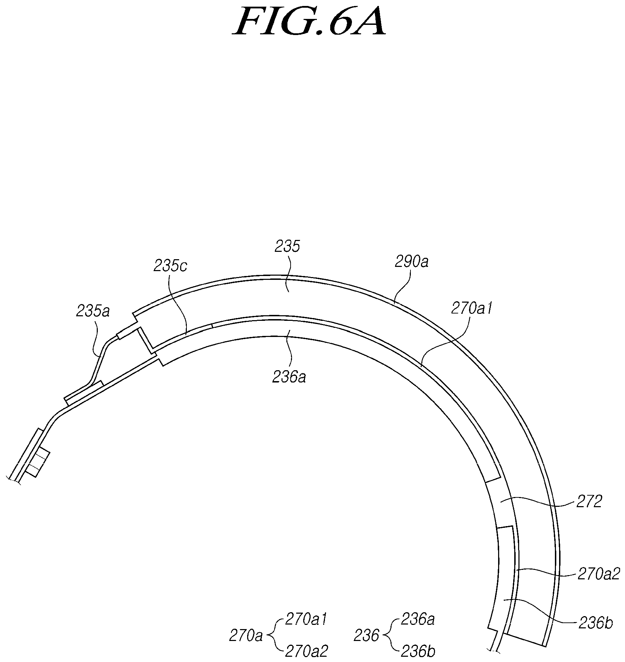

FIGS. 6A to 6C are views schematically illustrating an arrangement structure of some components provided inside a ring-shaped wearable electronic device according to an embodiment of the disclosure.

FIGS. 6A to 6C are views omitting the components corresponding to the molding member 250 and the frame 210 illustrated and described in connection with FIGS. 2 to 5, for convenience of description, in describing the arrangement structure of the compression member 270.

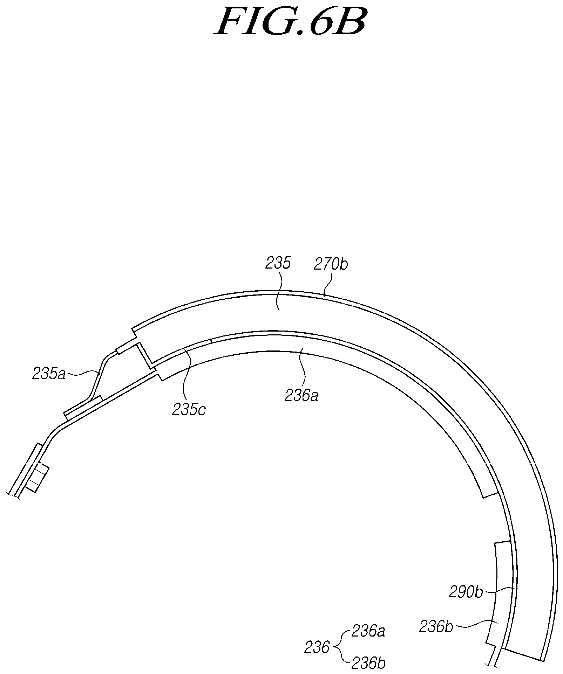

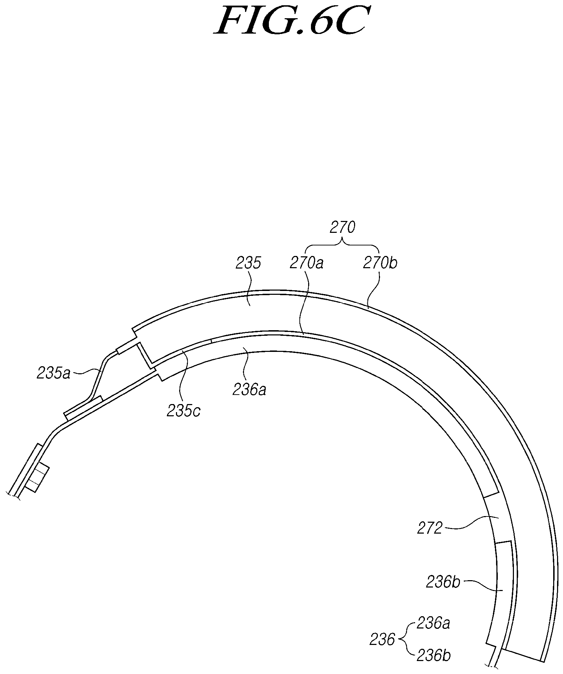

Specifically, referring to FIGS. 6A to 6C, FIG. 6A is a view illustrating a state in which the compression member 270a is disposed to contact the inner surface (or radially inner surface) of the battery 235. FIG. 6B is a view illustrating a state in which the compression member 270b is disposed to contact the outer surface (or radially outer surface) of the battery 235. FIG. 6C is a view illustrating a state in which the compression members 270a and 270b are disposed to contact the inner surface and the outer surface, respectively, of the battery 235.

Referring to FIG. 6A, the compression member 270a according to an embodiment may be disposed on a radial inner surface (e.g., a surface close to the hole 251) of the battery 235. Here, the inner surface of the battery 235 may refer to one surface of the battery 235 facing one or more FPCBs 236. In an embodiment, the compression member 270a may be positioned between the one or more FPCBs 236 and the battery 235.

According to an embodiment, the compression member 270a may extend along the inner surface of the battery 235. In an embodiment, the compression member 270a may be attached to a remaining area of the inner surface of the battery 235 except for an area to which the supporting member 235c is coupled. In an embodiment, the compression member 270a may include a partition 272 for dividing an area 270a1 where one side 236a of the one or more FPCBs 236 is attached and an area 270a2 where the other side 236b of the one or more FPCBs 236 is attached. In other words, the partition (or partition portion) 272 may separate area 270a1 from area 270a2. In an embodiment, the partition 272 may be designed to be thicker than the areas 270a1 and 270a2 where one or more FPCBs 236 are attached. In an embodiment, the partition 272 may protrude, or extend, from the radial inner surface (e.g., the surface close to the hole 251) of the compression member 270a (i.e. it may protrude or extend radially inwards).

In an embodiment, two opposite surfaces of the compression member 270a may contact at least a portion 236a and 236b of the one or more FPCBs 236 and the battery 235, respectively. In this case, two opposite radial side surfaces of the compression member 270a disposed in contact with the battery 235 and the one or more FPCBs 236, respectively, are surrounded by the battery 235 and the one or more FPCBs 236, respectively, not to be exposed to the outside. Accordingly, in the molding process for coupling the molding member 250 to the frame (e.g., the frame 210 of FIG. 3), the molding liquid does not penetrate two opposite radial side surfaces of the compression member 270a, and thus the compression performance of the compression member 270a may be maintained.

Further, in an embodiment, the adhesive member 290a may be attached to the outer surface of the battery 235. Here, the outer surface of the battery 235 is a surface opposite to the inner surface of the battery 235 described above and, although not specifically illustrated in the drawings, may refer to one surface of the battery 235 facing the frame (e.g., the frame 210 of FIG. 3). In this case, the battery 235 may be fixed to the inner circumferential surface of the frame 210 through the adhesive member 290a.

Referring to FIG. 6B, the compression member 270b according to an embodiment may be disposed on the radial outer surface of the battery 235, unlike that illustrated in FIG. 6A. In an embodiment, although not specifically illustrated, the compression member 270b may be positioned between the battery 235 and the frame 210. In an embodiment, the compression member 270b may extend along the outer surface of the battery 235. In an embodiment, the compression member 270b may be entirely attached to the outer surface of the battery 235. In an embodiment, two opposite surfaces of the compression member 270b may contact the battery 235 and the frame 210, respectively. In this case, two opposite radial side surfaces of the compression member 270b are surrounded by the battery 235 and the frame 210, respectively, not to be exposed to the outside. Accordingly, in the molding process for coupling the molding member 250 to the frame 210, the molding liquid does not penetrate two opposite radial side surfaces of the compression member 270b, and thus the compression performance of the compression member 270b may be maintained.

Further, in an embodiment, the adhesive member 290b may be attached to the inner surface of the battery 235. In this case, at least portions 236a and 236b of the one or more FPCBs 236 disposed on the side of the inner surface of the battery 235 may be fixed to the battery 235 through the adhesive member 290b.

Referring to FIG. 6C, the compression members 270a and 270b according to an embodiment may be disposed on two opposite surfaces (e.g. on two opposite radial side surfaces), respectively, of the battery 235, unlike those illustrated in FIGS. 6A and 6B. In an embodiment, the compression member 270 may include a first compression member 270a disposed on the radial inner surface of the battery 235 and a second compression member 270b disposed on the radial outer surface of the battery 235.

According to an embodiment, the first compression member 270a may be positioned between the one or more FPCBs 236 and the battery 235. In an embodiment, two opposite surfaces of the first compression member 270a may contact at least a portion of the one or more FPCBs 236 and the battery 235, respectively. In this case, two opposite radial side surfaces of the first compression member 270a are surrounded by the battery 235 and the one or more FPCBs 236, respectively, not to be exposed to the outside.

According to an embodiment, the second compression member 270b may be positioned between the battery 235 and the frame 210, although not specifically illustrated. In an embodiment, two opposite surfaces of the second compression member 270b may contact the battery 235 and the frame 210, respectively. In this case, two opposite radial side surfaces of the second compression member 270b are surrounded by the battery 235 and the frame 210, respectively, not to be exposed to the outside in the radial direction.

Further, comparing FIGS. 6A, 6B, and 6C, when the compression members 270 are provided on two opposite radial side surfaces of the battery 235 as illustrated in FIG. 6C, adhesive member 290a or 290b for coupling the battery 235 and the frame 210 or the battery 235 and one or more FPCBs 236, illustrated in FIGS. 6A and 6B, may be replaced by the compression members 270a and 270b and, as the respective compression members 270a and 270b contact the two opposite radial sides of the battery 235, the compression member 270 may maximally absorb the expanding portion of the battery 235 during battery swelling.

FIG. 7 is a perspective view illustrating an arrangement structure between some components provided inside a ring-shaped wearable electronic device according to an embodiment of the disclosure. For convenience of description, only part 236a of the one or more FPBC 236 structures disposed at the inner lower end of the battery 235 and the compression member 270c is illustrated with dashed lines, and a supporting member (e.g., the supporting member 235c of FIG. 3) of the battery 235 is omitted.

Referring to FIG. 7, one or more swelling holes 271 (which may also be described as windows, holes, apertures, voids, empty spaces, or empty volumes) may be provided inside the compression member 270c according to an embodiment. In an embodiment, the one or more swelling holes 271 may be provided in each of an area 270c1 where one side portion 236a of the one or more FPCBs 236 is attached and an area 270c2 where one side portion 236a of the one or more FPCBs 236 is attached. In an embodiment, the compression member 270c may have a rectangular band shape as a whole (e.g. it may extend around a rectangular perimeter, with a void in the middle), but the disclosure is not limited thereto. In this case, when the swelling of the battery 235 occurs, the expanding portion of the battery 235, which is inflated according to the battery swelling, may be received in a space (or volume, or void) formed by the swelling hole 271 of the compression member 270c.

According to an embodiment, the compression member 270c may also have a predetermined compression rate, like each of the compression members 270, 270a, and 270b described above with reference to FIGS. 2 to 4 or FIGS. 6A to 6C. Accordingly, in the compression member 270c, a portion of the remaining portion (e.g., the edge portion of the compression member 270c) other than the swelling hole 271, in which the molding liquid does not penetrate, has compression performance and may absorb the expanded battery volume during battery swelling.

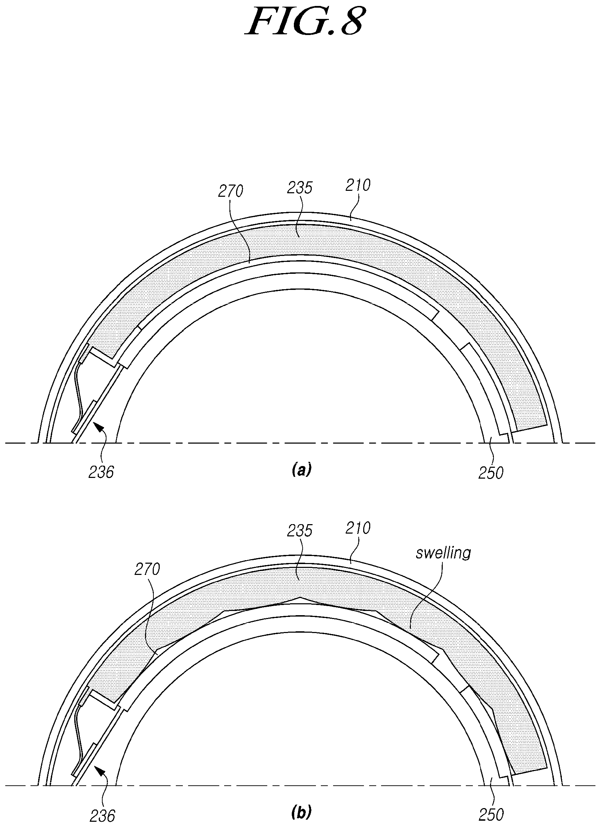

FIG. 8 is a view schematically illustrating a change in shape of a battery and a compression member when a battery swells in a ring-shaped wearable electronic device according to an embodiment of the disclosure.

FIG. 8 is a view illustrating a state in which the compression member 270 is compressed by the battery 235 during swelling of the battery 235 when the compression member 270 is positioned between the battery 235 and the FPCB 236. Specifically, FIG. 8(a) is a view illustrating a state of the battery 235 and the compression member 270 before the battery swells, and FIG. 8B is a view illustrating a state of the battery 235 and the compression member 270 after the battery swells.

Due to various causes such as overcharge/overdischarge of the battery 235, heat generation, exposure to a high-temperature environment, or aging of the battery 235, the electrolyte inside the battery 235 may vaporize and the battery 235 may swell due to the pressure. Specifically, when the battery 235 swells, as illustrated in FIG. 8(b), the electrolyte inside the battery 235 is vaporized, so that the battery 235 may swell in the inner radial direction and/or the outer radial direction of the battery 235, thereby expanding the volume of the battery 235.

As illustrated in FIG. 8(b), when the battery 235 swells while the compression member 270 is disposed between the battery 235 and the FPCB 236, the inside of the battery 235 swells due to the pressure of the vaporized electrolyte solution, expanding the overall volume of the battery 235. The compression member 270 attached to the battery 235 may be simultaneously compressed by the expanding portion of the battery 235. In other words, the compression member 270 is compressed by the amount corresponding to the expanded volume of the battery 235 during swelling, absorbing the expanded volume of the battery 235. Accordingly, the pressure applied by the expanded battery 235 to the molding member 250 adjacent to the battery 235 due to battery swelling may be reduced, thereby preventing cracks in the molding member 250 or reduction in the inner diameter of the wearable electronic device 200.

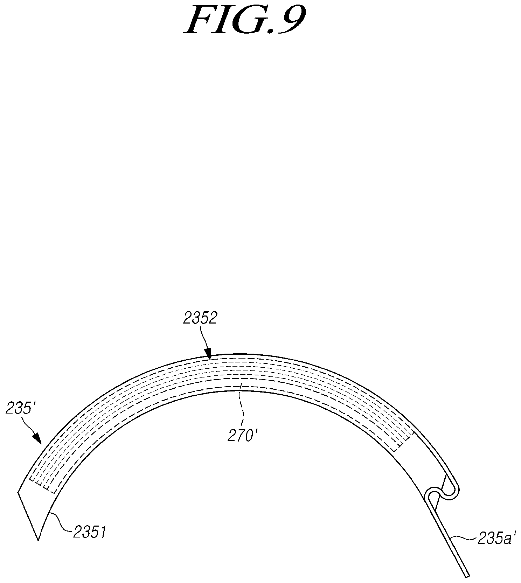

FIG. 9 is a plan view illustrating a structure of a battery of a ring-shaped wearable electronic device according to an embodiment of the disclosure.

According to an embodiment of the disclosure, as illustrated in FIG. 9, a compression member 270' may be disposed inside a battery 235'.

Referring to FIG. 9, in an embodiment, the battery 235' may include a battery housing 2351 forming an outer appearance of the battery 235', and a battery stack 2352 disposed inside the battery housing 2351 and in which a cathode, a separator, and an anode are sequentially and repeatedly stacked.

According to an embodiment, unlike the above-described compression members 270, 270a, and 270b disposed outside the housing of the battery 235, the compression member 270' may be disposed inside the battery housing 2351 of the battery 235'. In an embodiment, the compression member 270' may be positioned between the battery housing 2351 and the battery stack 2352, inside the battery 235'. In an embodiment, the compression member 270' may be disposed adjacent to any one of outermost edges on two opposite sides of the battery stack 2352 in the stacking direction. In this case, when the battery 235' swells, the compression member 270' is compressed by the pressure of the electrolytic solution vaporized inside the battery, absorbing the volume expansion of the battery 235'.

According to an embodiment of the disclosure, a wearable electronic device 200 may include a ring-shaped frame 210. The wearable electronic device 200 may include a battery 235 seated on (or over, or under, or supported by) the frame 210. The wearable electronic device 200 may include an electronic component 236 electrically connected to the battery 235. The wearable electronic device 200 may include a member 250 (e.g. a molding, or molded, member 250) coupled (or connected or attached) to the frame 210 to surround the battery 235 and the electronic component 236. In other words, the frame 210 and member 250 may, together, form a housing, and the battery 235 and electronic component 236 may be contained or encapsulated inside the housing (i.e. they may be accommodated in a volume inside the housing). The frame 210 may be described as a first housing portion, part, or component, and the member 250 may be described as a second housing portion, part, or component. The wearable electronic device may include a compression member 270a, 270b, or 270c (or at least one compression member, or a plurality of compression members) disposed to contact the battery 235 (or, in other words, to be in contact with the battery). The compression member may be configured so that a part thereof, in contact with an expanding portion of the battery 235, is compressible when the battery 235 swells. In other words, the at least one compression member may be configured to be compressed by an expanding portion of the battery when the battery (235) swells. The compression member 270a, 270b, or 270c may be positioned between the frame 210 and the molding member 250.

According to an embodiment of the disclosure, at least one compression member 270a, 270b, or 270c may be provided. The at least one compression member 270a, 270b, or 270c may be positioned in at least one of a space between the frame 210 and the battery 235 or a space between the battery 235 and the electronic component 236.

According to an embodiment of the disclosure, the (or each) compression member 270a, 270b, or 270c may include a hole 271 configured to receive at least one expanding portion of the battery 235 when the battery 235 swells.

According to an embodiment of the disclosure, a compression rate of the (or each) compression member 270a, 270b, or 270c may be substantially identical to or larger than an expansion rate of the battery 235 when the battery 235 swells.

According to an embodiment of the disclosure, an adhesive material may be coated on two opposite surfaces of the (or each) compression member 270a, 270b, or 270c facing the electronic component 236 and the battery 235, respectively, or facing the frame 210 and the battery 235, respectively.

According to an embodiment of the disclosure, the wearable electronic device 200 may further include an adhesive member 290a or 290b configured to attach the frame 210 and the battery 235 to each other or attach the battery 235 and the electronic component 236 to each other. The adhesive member 290a or 290b may be positioned in a space where the (or each) compression member 270a, 270b, or 270c is not positioned, of the space between the frame 210 and the battery 235 or the space between the battery 235 and the electronic component 236.

According to an embodiment of the disclosure, the (or each) compression member 270a, 270b, or 270c may be (or may comprise, or may consist of) at least one of polyurethane foam, rubber, elastomer, chloroprene, silicone, or neoprene.

According to an embodiment of the disclosure, the molding member 250 may be coupled to an inner circumferential surface of the frame 210 to configure an inner portion of the wearable electronic device 200.

According to an embodiment of the disclosure, the molding member 250 may be coupled to an outer circumferential surface of the frame 210 to configure an outer portion of the wearable electronic device 200.

According to an embodiment of the disclosure, the battery 235 may have a curvature substantially the same as a curvature of one surface of the frame 210 on which the battery 235 is mounted.

According to an embodiment of the disclosure, a wearable electronic device 200 may include a ring-shaped frame 210. The wearable electronic device 200 may include a battery 235' seated on the frame 210. The wearable electronic device 200 may include an electronic component 236 electrically connected to the battery 235'. The wearable electronic device 200 may include a molding member 250 coupled to the frame 210 to surround the battery 235' and the electronic component 236. The wearable electronic device 200 may include a compression member 270' (or at least one compression member) disposed inside the battery 235' and configured to be compressible, for example to offset a pressure generated inside the battery 235' when the battery 235' swells (and/or prevent distortion of a battery housing when pressure increases inside the battery housing).

According to an embodiment of the disclosure, the battery 235' may include a battery housing 2351 and a battery stack 2352 received inside the battery housing 2351. The compression member 270' may be positioned between the battery housing 2351 and the battery stack 2352 inside the battery 235'.

According to an embodiment of the disclosure, an adhesive material may be coated on two opposite surfaces of the compression member 270' facing the battery housing 2351 and the battery stack 2352, respectively.

According to an embodiment of the disclosure, a compression rate of the compression member 270' may be substantially identical to or larger than an expansion rate of the battery 235' when the battery 235' swells.

According to an embodiment of the disclosure, the compression member 270' may be formed of at least one of polyurethane foam, rubber, elastomer, chloroprene, silicone, or neoprene.

Another embodiment provides a wearable electronic device comprising electronic circuitry, a battery arranged to power the electronic circuitry (when in use), a housing defining an internal volume, and at least one compressible member, wherein the electronic circuitry, battery, and at least one compressible member are arranged in said internal volume, and the at least one compressible member is arranged between the battery and an internal surface of the housing so as to compress in response to an increase in volume or change of shape the battery and prevent said increase or change of shape from distorting the housing.

Another embodiment provides a wearable electronic device comprising electronic circuitry, a battery arranged to power the electronic circuitry (when in use), and a housing defining an internal volume, wherein the electronic circuitry and battery are arranged in said internal volume, and the battery comprises a battery housing and at least one compressible member inside the battery housing. The compressible member may be arranged to compress and prevent distortion of the battery housing, for example in response to a change in at least one internal property, state, or condition of the battery (or of a component of the battery) and/or in response to a change in a shape, size, or volume of at least one internal component of the battery. The compressible member(s) inside the battery housing may thus prevent the change or changes from distorting the battery housing and so from distorting the housing of the wearable electronic device.

## Claims

1. A wearable electronic device (200) comprising:
a ring-shaped frame (210);
a battery (235) seated on the frame (210);
an electronic component (236) electrically connected to the battery (235);
a molding member (250) coupled to the frame (210) to surround the battery (235) and the electronic component (236); and
a compression member (270a, 270b, 270c) disposed to contact the battery (235) and configured so that a part thereof, in contact with an expanding portion of the battery (235), is compressible when the battery (235) swells,
wherein the compression member (270a, 270b, 270c) is positioned between the frame (210) and the molding member (250).

2. The wearable electronic device of claim 1, wherein at least one compression member (270a, 270b, 270c) is provided, and
wherein the at least one compression member (270a, 270b, 270c) is positioned in at least one of a space between the frame (210) and the battery (235) or a space between the battery (235) and the electronic component (236).

3. The wearable electronic device of claim 1 or 2, wherein the compression member (270a, 270b, 270c) includes a hole (271) configured to receive the expanding portion of the battery (235) when the battery (235) swells.

4. The wearable electronic device of any one of claim 1 to 3, wherein a compression rate of the compression member (270a, 270b, 270c) is substantially identical to or larger than an expansion rate of the battery (235) when the battery (235) swells.

5. The wearable electronic device of any one of claim 2 to 4, wherein an adhesive material is coated on two opposite surfaces of the compression member (270a, 270b, 270c) facing the electronic component (236) and the battery (235), respectively, or facing the frame (210) and the battery (235), respectively.

6. The wearable electronic device of any one of claim 2 to 5, further comprising an adhesive member (290a, 290b) configured to attach the frame (210) and the battery (235) to each other or attach the battery (235) and the electronic component (236) to each other,
wherein the adhesive member (290a, 290b) is positioned in a space where the compression member (270a, 270b, 270c) is not positioned, of the space between the frame (210) and the battery (235) or the space between the battery (235) and the electronic component (236).

7. The wearable electronic device of any one of claim 1 to 6, wherein the compression member (270a, 270b, 270c) is at least one of polyurethane foam, rubber, elastomer, chloroprene, silicone, or neoprene.

8. The wearable electronic device of any one of claim 1 to 7, wherein the molding member (250) is coupled to an inner circumferential surface of the frame (210) to configure an inner portion of the wearable electronic device (200).

9. The wearable electronic device of any one of claim 1 to 7, wherein the molding member (250) is coupled to an outer circumferential surface of the frame (210) to configure an outer portion of the wearable electronic device (200).

10. The wearable electronic device of any one of claim 1 to 9, wherein the battery (235) has a curvature substantially the same as a curvature of one surface of the frame (210) on which the battery (235) is mounted.

11. A wearable electronic device (200) comprising:
a ring-shaped frame (210);
a battery (235') seated on the frame (210);
an electronic component (236) electrically connected to the battery (235');
a molding member (250) coupled to the frame (210) to surround the battery (235') and the electronic component (236); and
a compression member (270') disposed inside the battery (235') and configured to be compressible to offset a pressure generated inside the battery (235') when the battery (235') swells.

12. The wearable electronic device of claim 11, wherein the battery (235') includes a battery housing (2351) and a battery stack (2352) received inside the battery housing (2351), and
wherein the compression member (270') is positioned between the battery housing (2351) and the battery stack (2352) inside the battery (235').

13. The wearable electronic device of claim 12, wherein an adhesive material is coated on two opposite surfaces of the compression member (270') facing the battery housing (2351) and the battery stack (2352), respectively.

14. The wearable electronic device of any one of claim 11 to 13, wherein a compression rate of the compression member (270') is substantially identical to or larger than an expansion rate of the battery (235') when the battery (235') swells.

15. The wearable electronic device of any one of claim 11 to 14, wherein the compression member (270') is formed of at least one of polyurethane foam, rubber, elastomer, chloroprene, silicone, or neoprene.
